(19) Europäisches Patentamt
European Patent Office
Office européen des brevets

(11) Veröffentlichungsnummer: **0 015 562**
**B1**

# (12) EUROPÄISCHE PATENTSCHRIFT

(45) Veröffentlichungstag der Patentschrift: **18.11.81**

(51) Int. Cl.³: **C 07 D 311/66**

(21) Anmeldenummer: **80101153.7**

(22) Anmeldetag: **07.03.80**

## (54) Verfahren zur Herstellung von Chromanderivaten.

(30) Priorität: **12.03.79 DE 2909601**

(43) Veröffentlichungstag der Anmeldung: **17.09.80 Patentblatt 80/19**

(45) Bekanntmachung des Hinweises auf die Patenterteilung: **18.11.81 Patentblatt 81/46**

(84) Benannte Vertragsstaaten: **BE CH DE FR GB IT NL**

(56) Entgegenhaltungen:
**CH-A5-565 782**
**DE-A-2 364 141**
**DE-A1-2 456 159**
**US-A-4 110 346**

(73) Patentinhaber: **BASF Aktiengesellschaft, Carl-Bosch-Strasse 38, D-6700 Ludwigshafen (DE)**

(72) Erfinder: **Horner, Michael, Dr,, Pfalzgrafenstrasse 15B, D-6730 Neustadt (DE)**
Erfinder: **Nissen, Axel, Dr,, Panoramastrasse 51, D-6906 Leimen (DE)**

**EP 0 015 562 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Verfahren zur Herstellung von Chromanderivaten

Die vorliegende Erfindung betrifft ein neues Verfahren zur Herstellung von Chromanderivaten der allgemeinen Formel I

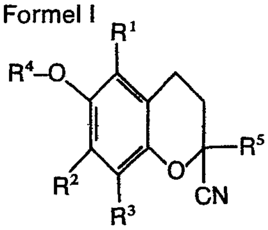

I

in welcher die Reste $R^1$–$R^3$ Wasserstoff oder Alkylgruppen mit 1–8 C-Atomen, $R^4$ Wasserstoff oder eine Alkyl-oder Acylgruppe mit 1–8 C-Atomen und $R^5$ eine Alkylgruppe mit 1–8 C-Atomen bedeuten.

Aus der DE-OS 2 364 141 ist es bekannt, das 6-Hydroxy-2-cyano-2,5,7,8-tetramethylchroman, ausgehend von Trimethylhydrochinon und Vinylmethylketon, in einer fünfstufigen Synthese herzustellen, wie aus dem folgenden Reaktionsschema hervorgeht.

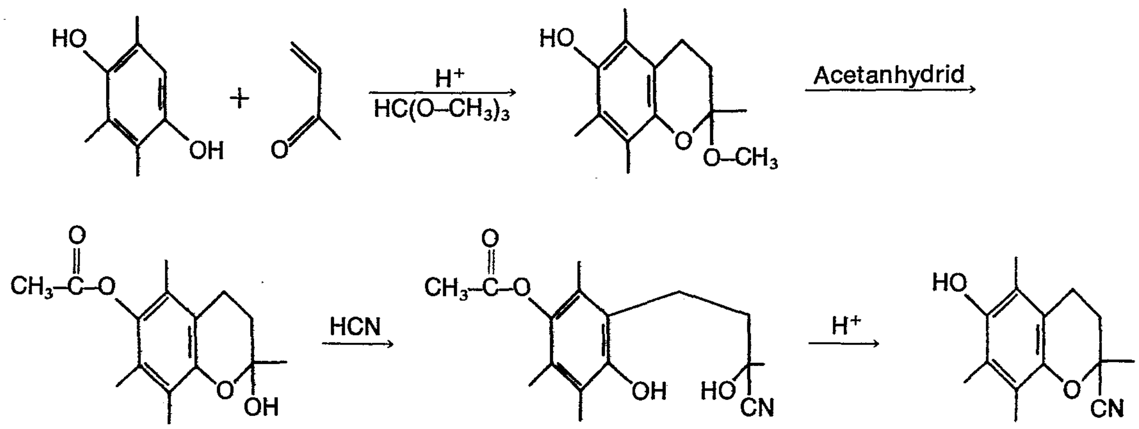


Ferner ist in den Beispielen 52 und 54 dieser DE-OS die Herstellung von 2-Cyano-6-hydroxy-2,5,7,8-tetramethylchroman in einer zweistufigen Synthese beschrieben, indem man Trimethylhydrochinon zunächst mit Dimethylamin zum 2-Dimethylaminomethyl-3,5,6-Trimethylhydrochinon umsetzt und die letztgenannte Verbindung mit Methacrylnitril in das gewünschte Chromanderivat überführt. Hierbei sind jedoch nur Ausbeuten von etwa 20% zu erzielen, so dass diese Verfahrensweise für technische Zwecke nicht in Betracht kommt.

Da die aus der DE-OS 2 364 141 bekannten Synthesewege zur Herstellung der Chromanderivate I somit entweder evident umständlich oder hinsichtlich der Ausbeuten unbefriedigend sind, lag der Erfindung die Aufgabe zugrunde, die für die Herstellung von Antioxidantien wichtigen Chromanderivate besser zugänglich zu machen.

Es wurde gefunden, dass man Chromanderivate der allgemeinen Formel I

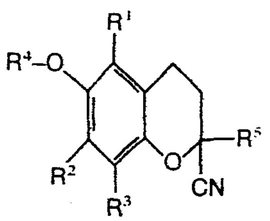

I

in welcher $R^1$–$R^3$ Wasserstoff oder Alkylgruppen mit 1–8 C-Atomen, $R^4$ Wasserstoff oder eine Alkyl- oder Acylgruppe mit 1–8 C-Atomen und $R^5$ eine Alkylgruppe mit 1–8 C-Atomen bedeuten, in einer bemerkenswerten einstufigen Reaktion auf wirtschaftliche Weise erhält, wenn man ein Hydrochinon der allgemeinen Formel II

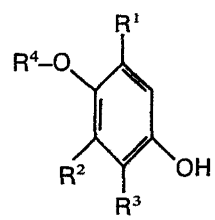

II

mit 10–300 Mol-%, bezogen auf II, einer Lewissäure oder eines Lewissäureadduktes mit einer schwachen Lewisbase oder eines Lewissäureadduktes einer Protonensäure in Gegenwart eines inerten Lösungsmittels mit einem Nitril der allgemeinen Formel III

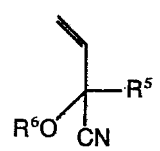

III

in welcher $R^6$ Wasserstoff oder eine Acyl- oder Alkylgruppe mit 1–4 C-Atomen bedeutet, oder mit Verbindungen umsetzt, aus welchen III unter den Reaktionsbedingungen in situ entsteht.

Unter den definitionsgemässen Ausgangsverbindungen II werden solche bevorzugt, in denen die Summe der C-Atome in den Alkylresten $R^1$–$R^3$ 3–10 beträgt. Von besonderer Bedeutung sind hierbei das Trimethylhydrochinon sowie ferner Hydrochinonderivate, in denen einer der Reste $R^1$–$R^3$ einen langkettigen Alkylrest und die anderen

Wasserstoff oder Methylgruppen bedeuten. Weiterhin kommen beispielsweise das Hydrochinon, das tert.-Butylhydrochinon und das 2,3- und 2,6-Dimethylhydrochinon in Betracht.

Geht man von solchen definitionsgemässen Hydrochinonen II, in denen die 1-Hydroxylgruppe geschützt, d. h. veräthert oder verestert ist ($R^4 \neq H$), so erhält man neben den entsprechenden, in 6-Stellung substituierten Chromanderivaten I stets auch die freie 6-Hydroxy-Verbindung. Beide Verfahrensprodukte sind jedoch aufgrund ihres unterschiedlichen Löslichkeitsverhaltens leicht zu trennen. Von den geschützten Hydrochinonen geht man vor allem dann aus, wenn die 3- und 6-Stellung des Benzolrings unsubstituiert sind und eine Bisaddition von III unterdrückt werden soll. Häufig ist die 1-Hydroxylgruppe von II bereits von der Synthese her geschützt; in diesem Falle ist es nicht erforderlich, die Schutzgruppe vor der weiteren Umsetzung abzuspalten. Schliesslich kann der Schutz der 6-Hydroxylgruppen in I von Vorteil sein, wenn man mit I weitere Umsetzungen vornehmen will.

Unter den definitionsgemässen Ausgangsverbindungen III sind diejenigen hervorzuheben, in denen $R^5$ eine Alkylgruppe mit 1–4 C-Atomen ist, wobei der Methylgruppe eine besondere Bedeutung zukommt. Diese Verbindungen sind bekannt oder in bekannter Weise erhältlich.

Anstelle der Nitrile III kann man auch Verbindungen einsetzen, aus denen III unter den Reaktionsbedingungen in situ entsteht, darunter vor allem Nitrile der allgemeinen Formel IIIa

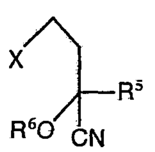 IIIa

in welcher X Halogen, die Hydroxylgruppe oder eine Alkoxy- oder Acyloxygruppe mit 1–4 C-Atomen bedeutet.

Da die Cyanhydrine ($R^6 = H$) III und IIIa ohnehin unter schwach sauren Bedingungen aus den Carbonylverbindungen IIIb und IIIc

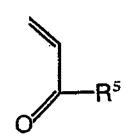 IIIb ; 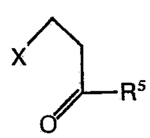 IIIc

und einer Cyanverbindung IV

Y–CN IV

in der Y Wasserstoff, das Äquivalent eines Metallkations oder eine Trialkylsilylgruppe bedeutet, entstehen, ist es häufig zweckmässig, bei der Synthese von I statt von III von einem äquimolaren oder annähernd äquimolaren Gemisch aus IIIb oder IIIc und IV auszugehen, wodurch sich der Verfahrensschritt der getrennten Herstellung von III oder IIIa einsparen lässt.

Weiterhin bilden sich die Verbindungen III aus IV und Enoläthern und Ketalen der Formeln IIId–g

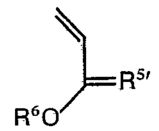 IIId ; 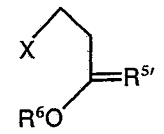 IIIe

$R^{5'}$ ist der dem Alkylrest $R^5$ entsprechende Alkylidenrest $R^6 \neq H$

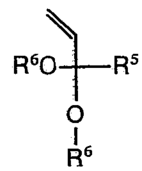 III f; 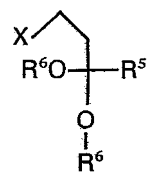 IIIg

$R^6 \neq H$

In wirtschaftlicher Hinsicht sind die Verbindungen IIId–g als Einsatzstoffe für das erfindungsgemässe Verfahren jedoch normalerweise von untergeordneter Bedeutung.

Verbindungen des Typs III und IIIa mit $R^6 \neq H$ sind in bekannter Weise erhältlich, z. B. durch Alkylierung oder Acylierung der freien Alkohole. Als Reste $R^6$ kommen vornehmlich die Methylgruppe, die Äthylgruppe und die Acetylgruppe in Betracht.

Den Ausgangsverbindungen III, IIIb, IIId und IIIf ist die für die Addition an II erforderliche Vinylgruppe gemeinsam, die nach Art einer Friedel-Crafts-Reaktion reagiert. Anstelle der Vinylgruppe können daher auch die Gruppierungen $-CH_2-CH_2-X$ stehen, wie sie sich für Friedel-Crafts-Alkylierungen eignen, so dass man auch von den Verbindungen IIIa, IIIe und IIIg ausgehen kann. Geeignete Reste X sind vornehmlich Chlor und Brom, die Hydroxylgruppe, die Methoxygruppe und die Acetoxygruppe.

Unter den genannten Verfahrensweisen wird die Umsetzung von II mit einem Cyanhydrin III ($R^6 = H$) in der Regel bevorzugt, weil sie mit den besten Ausbeuten verbunden ist. Die übrigen Methoden können sich dagegen empfehlen, wenn die betreffenden Ausgangsverbindungen besonders leicht zugänglich sind.

Vorzugsweise setzt man II und III bzw. deren Vorstufen in äquimolaren Mengen ein, jedoch kann es sich zur Beschleunigung der Reaktion empfehlen, III in einem bis zu 1,2-molaren Überschuss zu verwenden. Grössere Überschüsse bringen in aller Regel keine Vorteile mehr.

Wesentlich für das erfindungsgemässe Verfahren ist die Verwendung einer Lewissäure für die Verknüpfung von III mit II. Die Menge der Lewissäure, bezogen auf II, beträgt 10–300, vorzugsweise 30–120 Mol-%. Sofern X die Hydroxylgruppe bedeutet, ist zu berücksichtigen, dass 1 Mol der Lewissäure bereits von der OH-Gruppe verbraucht wird.

Als katalytisch wirksame Verbindungen eignen sich

– die Lewissäure selber, beispielsweise Borhalogenide wie $BF_3$, $BCl_3$ und $BJ_3$, Aluminiumhalogenide wie $AlCl_3$, $AlBr_3$ und $AlJ_3$, Zinntetrahalogenide wie $SnCl_4$, $SnBr_4$ und $SnJ_4$, Zinkhalogenide, wie $ZnCl_2$, $ZnBr_2$ und $ZnJ_2$, sowie ferner Eisendihalogenide;

– die Addukte von Lewissäuren mit Lewisbasen, die eine geringere Basenstärke haben als die Ausgangsverbindungen II, also z. B. die Addukte der vorgenannten Metallhalogenide mit Äthern wie Dimethyläther, Diäthyläther und Tetrahydrofuran, Estern wie Äthylacetat, Nitrilen wie Acetonitril und Benzonitril, Lactonen wie $\gamma$-Butyrolacton, Nitroverbindungen wie Nitromethan und Nitrobenzol und Ketonen wie Aceton und Acetophenon. Die Verwendung dieser Addukte bietet den Vorteil einer besseren verfahrenstechnischen Handhabung, da sie wegen ihrer Löslichkeit in organischen Medien leichter dosierbar sind; ausserdem gestatten es die Addukte meist von vornherein, in homogener Phase zu arbeiten;
– die Addukte von Lewissäuren mit Protonensäuren, z. B. mit Mineralsäuren wie Phosphorsäure, Schwefelsäure und Salzsäure. Diese Addukte, die sich als Komplexsäuren vom Typ

$$HCl + BCl_3 \longleftrightarrow H[BCl_4]$$

formulieren lassen, beschleunigen die erfindungsgemässe Umsetzung häufig stärker als die ihnen zugrunde liegenden Lewissäuren allein.

Unter den Lewissäuren werden Bortrifluorid und Bortrifluorid-Diäthylätherat bevorzugt.

Als inerte Lösungsmittel eignen sich allgemein aprotische Flüssigkeiten, deren Donizität höchstens 20 ist. Der Begriff der Donizität ist in der Arbeit von V. Gutmann, Angewandte Chemie, Band 82 (1972), S. 858, definiert. Er ist ein Ausdruck für die Affinität zu Lewissäuren und bedeutet für den Fall der vorliegenden Massgabe, dass die Lösungsmittel mit den Lewissäuren keine stabilen Anlagerungskomplexe bilden sollen. Geeignete Lösungsmittel sind demgemäss z. B. Dichlormethan, 1,2-Dichloräthan, Chloroform, Benzol, Toluol, Chlorbenzol, Nitromethan, Nitrobenzol, Benzonitril, Acetonitril, Cyclohexan und Mischungen dieser Lösungsmittel.

Unter den genannten Lösungsmitteln sind Gemische von Toluol oder Dichlormethan mit Nitromethan besonders gut geeignet. Die Funktion der Lösungsmittel ist es, dass mindestens ein Teil der eingesetzten Reaktionspartner in homogener Lösung gehalten wird. Die Menge der Lösungsmittel kann in weiten Grenzen schwanken, beträgt im allgemeinen jedoch 1–10 kg pro kg II.

Die Reaktionstemperaturen liegen vorzugsweise zwischen (–50) und 100 °C, vor allem zwischen (–40) und 60 °C. Da die Reaktion unter Umständen sehr heftig verlaufen kann, was zu einer vermehrten Bildung unerwünschter Produkte führen kann, empfiehlt es sich, bei niedrigen Temperaturen zu beginnen und die Temperatur dann allmählich zu erhöhen. Eine zu heftige Reaktion kann auch durch allmähliche Zugabe der Reaktionspartner vermieden werden.

Da die Reaktion bei Normaldruck ausgeführt werden kann, besteht in aller Regel kein Grund, sie unter erniedrigtem oder erhöhtem Druck vorzunehemen.

Sonstige verfahrenstechnische Besonderheiten sind nicht zu beachten, d. h. man kann die Umsetzung nach den üblichen präparativen Techniken vornehmen, indem man z. B. eine Lösung von III bzw. den Vorstufen von III zusammen mit IV vorlegt und hierzu gleichzeitig allmählich eine Lösung von II sowie die Lewissäure oder eine Lösung der Lewissäure oder eines der genannten Lewissäure-Addukte zugibt. Da manche Cyanhydrine III leicht instabil sind, können diese mit etwas Säure, z. B. Phosphorsäure, stabilisiert werden.

Die Aufarbeitung der Reaktionsgemische kann nach den üblichen Methoden erfolgen, wie sie für Friedel-Crafts-Reaktionen bekannt sind, etwa indem man das Gemisch mit Wasser und gegebenenfalls zusätzlich mit Äther versetzt und die Lösungsmittel von der getrockneten organischen Phase abdestilliert. Das so erhältliche Rohprodukt, welches I in etwa 70–90%iger Ausbeute enthält, kann gewünschtenfalls durch Umkristallisation, z. B. aus Aceton/Wasser, gereinigt werden.

Die Verbindungen I können in an sich bekannter Weise durch Hydrolyse in die Chroman-Carbonsäuren V

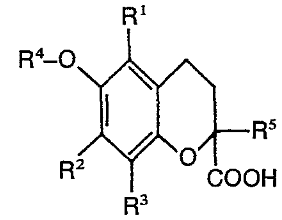

V

überführt werden.

Die Verbindungen V, darunter besonders die 6-Hydroxy-2,5,7,8-tetramethylchroman-2-carbonsäure Va

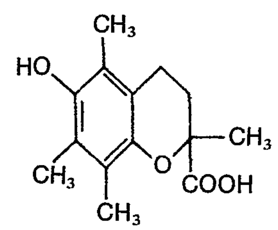

Va

sind bekanntermassen wichtige Antioxidantien für organische Materialien wie Fette und Öle sowie pharmazeutische und kosmetische Zubereitungen.

Beispiel 1

Eine Lösung aus 33,6 g (0,338 Mol) Methylvinylketon-Cyanhydrin, das mit 1,3 g Phosphorsäure als Stabilisierungsmittel vermischt war, 63 g Toluol und 34 g Nitromethan wurde im Laufe einer Stunde unter Stickstoffatmosphäre bei 0–5 °C mit einer Suspension aus 180 g Toluol, 51,4 g (0,338 Mol) Trimethylhydrochinon und 23,0 g (0,338 Mol) $BF_3$ versetzt. Die Suspension wurde anschliessend noch eine Stunde bei (–5) °C gerührt, dann im Laufe von drei Stunden auf 20 °C erwärmt und weitere zehn Stunden bei dieser Temperatur gehalten.

Dieses Reaktionsgemisch, eine rote Lösung mit einem farblosen nadelförmigen Niederschlag, wurde anschliessend mit 200 ml Wasser und 500 ml Äther versetzt und wie üblich aufgearbeitet. Die Menge des lösungsmittelfreien organischen Rückstandes betrug 76 g, wobei nach gaschromatographischer Analyse 66,9 g auf das 6-Hydroxy-2-cyano-2,5,7,8-tetramethylchroman und 7,6 g auf nichtumgesetztes Trimethylhydrochinon entfielen.

Die Umkristallisation aus Aceton/Wasser lieferte das reine Chromanderivat in 81%iger Ausbeute, bezogen auf eingesetztes Trimethylhydrochinon.

Das gleiche Ergebnis wurde erzielt, indem die Trimethylhydrochinon-Suspension vorgelegt, dann mit dem $BF_3$ und anschliessend mit dem Cyanhydrin versetzt wurde.

Beispiel 2

Auf die im letzten Absatz von Beispiel 1 beschriebene Weise, jedoch mit 0,338 Mol $BF_3$-Diäthylätherat anstelle des freien $BF_3$ wurde das 6-Hydroxy-2-cyano-2,5,7,8-tetramethylchroman in 79%iger Ausbeute hergestellt.

Beispiel 3

Nach der Arbeitsweise des letzten Absatzes von Beispiel 1, jedoch mit 0,338 Mol Zinntetrachlorid anstelle des $BF_3$, fiel das reine Chromderivat in 75%iger Ausbeute an.

Beispiel 4

Nach der Arbeitsweise des letzten Absatzes von Beispiel 1, jedoch unter Verwendung von wasserfreiem Methylenchlorid anstelle von Toluol als Lösungsmittel und von 0,338 Mol Aluminiumtrichlorid anstelle von $BF_3$ fiel das 6-Hydroxy-2-cyanochroman in 64%iger Ausbeute an.

Beispiel 5

Auf die im letzten Absatz von Beispiel 1 beschriebene Weise, jedoch mit 0,338 Mol wasserfreiem Zinkchlorid anstelle von $BF_3$ fiel das reine 6-Hydroxy-2-cyanochroman in 55%iger Ausbeute neben 26% nicht umgesetztem Trimethylhydrochinon an.

Beispiel 6

Nach der Arbeitsweise des letzten Absatzes von Beispiel 1, jedoch mit 0,338 Mol Zinkjodid anstelle von $BF_3$ wurde das 6-Hydroxy-2-cyanochroman in 62%iger Ausbeute hergestellt. Daneben wurden 20% nicht umgesetztes Trimethylhydrochinon isoliert.

Beispiel 7

Zu 45,0 g (0,338 Mol) $AlCl_3$ wurden im Laufe von 30 min bei 0 °C 15 ml Methylenchlorid und 40 ml Nitromethan gegeben. Die Reaktionsmischung wurde auf −20 °C gekühlt und danach im Laufe von 45 min mit 25,7 g (0,169 Mol) Trimethylhydrochinon, 19,4 g (0,169 Mol) 4-Hydroxybutan-2-on-cyanhydrin, das mit 1 g Phosphorsäure stabilisiert war, versetzt und 1 Stunde bei (−20) °C nachgerührt. Danach wurde die Temperatur innerhalb von 2 Stunden auf 25 °C erhöht. Nach weiterer 10stündiger Reaktionszeit bei 25 °C und anschliessender Aufarbeitung analog Beispiel 1 wurde das reine 6-Hydroxy-2-cyanochroman in 69%iger Ausbeute isoliert.

Beispiel 8

Nach dem im letzten Absatz in Beispiel 1 beschriebenen Verfahren wurden 23,3 g (0,169 Mol) 2,6-Dimethylhydrochinon in Gegenwart von 11,5 g (0,169 Mol) $BF_3$ mit 16,6 g (0,169 Mol) Methylvinylketoncyanhydrin, welches mit 1 g Phosphorsäure stabilisiert war, in 125 ml Toluol und 17 ml Nitromethan umgesetzt. Die übliche Aufarbeitung lieferte das 6-Hydroxy-2,5,7-trimethyl-2-cyanochroman in Form von farblosen Kristallen in 79%iger Ausbeute; Smp. 135 °C.

Beispiel 9

Nach dem im letzten Absatz von Beispiel 1 beschriebenen Verfahren wurden 57,4 g (0,34 Mol) Trimethylhydrochinon in Gegenwart von 24 g (0,34 Mol) $BF_3$ mit 19,9 g (0,34 Mol) Isopropylvinylketoncyanhydrin, das mit 1 g Phosphorsäure stabilisiert war, umgesetzt. Die übliche Aufarbeitung lieferte das 6-Hydroxy-5,7,8-trimethyl-2-isopropyl-2-cyanochroman als farblose Kristalle in 77%iger Ausbeute; Smp. 132 °C.

Beispiel 10

Nach dem im letzten Absatz von Beispiel 1 beschriebenen Verfahren, jedoch unter Verwendung von Methylenchlorid anstelle von Toluol wurden 56,0 g (0,338 Mol) tert.-Butylhydrochinon mit 33,6 g (0,338 Mol) Methylvinylketoncyanhydrin, das mit 1 g Phosphorsäure stabilisiert war, umgesetzt. Nach Chromatographie des Rohproduktes an Kieselgel mit Toluol/Äthylacetat fiel das 6-Hydroxy-7-tert.-butyl-2-methyl-2-cyanochroman in 30%iger Ausbeute an; farblose Kristalle vom Smp. 153–155 °C.

Daneben wurden, unter Entalkylierung des tert.-Butylhydrochinons, 22% 6-Hydroxy-2-methyl-2-cyanochroman erhalten; farblose Kristalle vom Smp. 146–148 °C.

Beispiel 11

Analog Beispiel 7 wurden 18,6 g (0,169 Mol) Hydrochinon mit 16,6 g (0,169 Mol) Methylvinylketoncyanhydrin, das mit 1 g Phosphorsäure stabilisiert war, in Gegenwart von 22,4 g (0,169 Mol) $AlCl_3$ umgesetzt. Nach der üblichen Aufarbeitung des Reaktionsgemisches ergab die GC-Analyse mit Toluol/Äthylacetat 22% 6-Hydroxy-2-methyl-2-cyanochroman; farblose Kristalle vom Smp. 146–148 °C.

Beispiel 12

Analog Beispiel 1, letzter Absatz, wurden 25,0 g (0,129 Mol) 4-Acetoxy-2,3,5-trimethylphenol mit 12,3 g (0,129 Mol) Methylvinylketoncyanhydrin, das mit Phosphorsäure stabilisiert war, mit 13,3 g (0,129 Mol) $BF_3$ umgesetzt. Die übliche Aufarbeitung des Reaktionsgemisches lieferte gemäss GC-Analyse 25% 6-Acetoxy-2,5,7,8-tetramethyl-2-cyanochroman; farblose Kristalle, Smp. 149–151 °C. Daneben fielen 15% 6-Hydroxy-2,5,7,8-tetramethyl-2-cyanochroman an.

Beispiel 13

Zu einer Lösung aus 9,7 g (0,36 Mol) flüssiger Blausäure und 34 g Nitromethan wurden unter

Rühren während 5 min 23,7 g (0,338 Mol) Methylvinylketon und danach zunächst bei (–20) °C und dann bei (–25) °C langsam 0,5 g Triäthylamin gegeben. Danach wurde das Reaktionsgemisch 1,5 Stunden bei (–20) °C gehalten. Nach Ansäuern des Reaktionsgemisches mit 1 g Phosphorsäure wurden 51,4 g (0,338 Mol) Trimethylhydrochinon und 180 g Toluol zugegeben und anschliessend wurden bei (–10) °C während 1,5 Stunden 23,0 g (0,338 Mol) $BF_3$ eingeleitet. Nach einstündigem Rühren bei (–5) °C wurde auf Raumtemperatur erhöht und 5 Stunden gerührt. Die Aufarbeitung analog Beispiel 1 erbrachte das reine 2-Cyanochroman in 78%iger Ausbeute.

**Patentansprüche**

1. Verfahren zur Herstellung von Chromanderivaten der allgemeinen Formel I

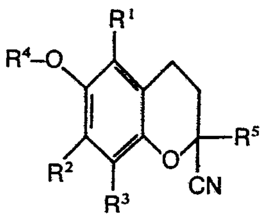 I

in welcher $R^1$–$R^3$ Wasserstoff oder Alkylgruppen mit 1–8 C-Atomen, $R^4$ Wasserstoff oder eine Alkyl- oder Acylgruppe mit 1–8 C-Atomen und $R^5$ eine Alkylgruppe mit 1–8 C-Atomen bedeuten, dadurch gekennzeichnet, dass man ein Hydrochinon der allgemeinen Formel II

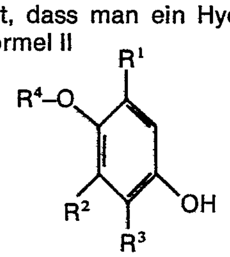 II

mit 10–300 Mol-%, bezogen auf II, einer Lewissäure oder eines Lewissäureadduktes mit einer schwachen Lewisbase oder eines Lewissäureadduktes mit einer Protonensäure in Gegenwart eines inerten Lösungsmittels mit einem Nitril der allgemeinen Formel III

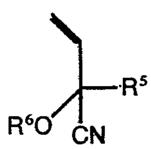 III

in welcher $R^6$ Wasserstoff oder eine Acyl- oder Alkylgruppe mit 1–4 C-Atomen bedeutet, oder mit Verbindungen umsetzt, aus welchen III unter den Reaktionsbedingungen in situ entsteht.

2. Verfahren nach Anspruch 1, dadurch gekennzeichnet, dass man als Verbindung, aus der III unter den Reaktionsbedingungen in situ entsteht, ein Nitril der allgemeinen Formel IIIa einsetzt

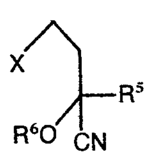 IIIa

in welcher X Halogen, die Hydroxylgruppe oder eine Alkoxy-oder Acyloxygruppe mit 1–4 C-Atomen bedeutet.

3. Verfahren nach Anspruch 1, dadurch gekennzeichnet, dass man als Verbindungen, aus denen III unter den Reaktionsbedingungen in situ entsteht ein äquimolares oder annähernd äquimolares Gemisch aus einer Cyanverbindung der allgemeinen Formel IV

$$Y\text{–}CN \qquad IV$$

in der Y für Wasserstoff, das Äquivalent eines Metallkations oder eine Trialkylsilylgruppe steht und einer Carbonylverbindung IIIb oder IIIc

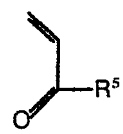 IIIb ; 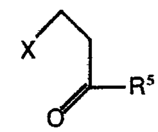 IIIc

einsetzt.

**Claims**

1. A process for the preparation of a chromane derivative of the general formula I

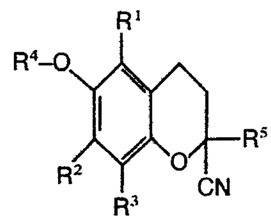 I

where $R^1$, $R^2$ and $R^3$ are hydrogen or alkyl of 1 to 8 carbon atoms, $R^4$ is hydrogen or alkyl or acyl of 1 to 8 carbon atoms and $R^5$ is alkyl of 1 to 8 carbon atoms, characterized in that a hydroquinone of the general formula II

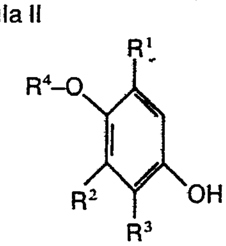 II

is reacted with 10–300 mole-%, based on II, of a Lewis acid or of a Lewis acid adduct with a weak Lewis base or of a Lewis acid adduct of a proton-donating acid, and with a nitrile of the general formula III

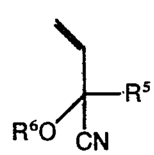 III

where $R^6$ is hydrogen or acyl or alkyl of 1 to 4 carbon atoms, or with a compound from which III is formed in situ under the reaction conditions, in the presence of an inert solvent.

2. A process as claimed in claim 1, characterized in that a nitril of the general formula IIIa

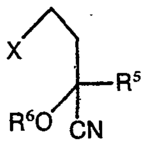

IIIa

where X is halogen, hydroxyl or alkoxy or acyloxy of 1 to 4 carbon atoms is used as the compound from which III is formed in situ under the reaction conditions.

3. A process as claimed in claim 1, characterized in that an equimolar or about equimolar mixture of a cyano compound of the general formula IV

Y–CN IV

where Y is hydrogen, the equivalent of a metal cation, or a trialkylsilyl group, and a carbonyl compound IIIb or IIIc

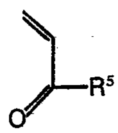 IIIb ; 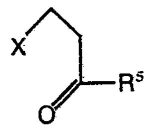 IIIc

is used as the compound from which III is formed in situ under the reaction conditions.

**Revendications**

1. Procédé de préparation de dérivés du chromane de la formule générale I

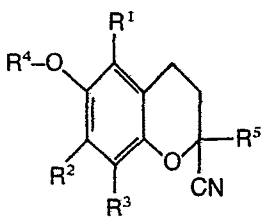 ,

I

dans laquelle $R^1$ à $R^3$ désignent des atoms d'hydrogène ou des radicaux alcoyle en $C_1$ à $C_8$, $R^4$ représente un atome d'hydrogène ou un groupe alcoyle ou acyle en $C_1$ à $C_8$ et $R^5$ un radical alcoyle en $C_1$ à $C_8$, caractérisé en ce que l'on fait réagir une hydroquinone de la formule générale II

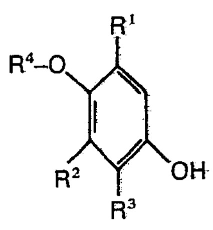

II

en présence d'un solvant inerte avec 10 à 300% molaires, par rapport à II, d'un acid de Lewis ou d'un produit d'addition d'un acide de Lewis et d'une base de Lewis faible ou d'un produit d'addition d'un acid de Lewis et d'un acid protonique et un nitrile de la formule générale III

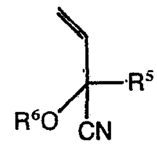 ,

III

dans laquelle $R^6$ représente un atome d'hydrogène ou un groupe acyle ou alcoyle en $C_1$ à $C_4$, ou avec des composés capables de former in situ, dans les conditions réactionnelles, un nitrile III.

2. Procédé suivant la revendication 1, caractérisé en ce que l'on emploie comme composé susceptible de former in situ, dans les conditions réactionnelles, un nitrile III, un nitrile de la formule générale IIIa

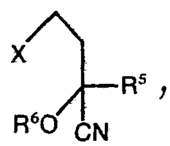 ,

IIIa

dans laquelle X désigne un atome d'halogène, un radical oxhydryle ou un groupe alcoxy ou acyloxy en $C_1$ à $C_4$.

3. Procédé suivant la revendication 1, caractérisé en ce que l'on emploie comme composés susceptibles de former in situ, dans les conditions réactionnelles, un nitrile III, un mélange équimolaire ou approximativement équimolaire d'un composé cyanique de la formule générale IV

Y–CN , IV

dans laquelle Y représente un atome d'hydrogène, l'équivalent d'un cation de métal ou un groupe trialcoyl-silyle, et d'un composé carbonylé IIIb ou IIIc.

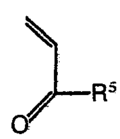 IIIb ; 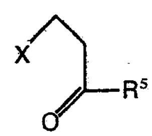 IIIc .